# EUROPEAN PATENT APPLICATION

(11) **EP 1 529 840 A1**
(43) Date of publication of application: **11.05.2005**
(21) Application number: 03025181.3
(22) Date of filing: 04.11.2003
(51) Int. Cl.: C12N 15/10, C07H 1/08

(54) **A rapid and low cost method for isolating nucleic acid**

(71) Applicant: Qiagen GmbH, 40724 Hilden (DE)
(72) Inventor: Himmelreich, Ralf, 40764 Langenfeld (DE); Fritz, Claudia, 51061 Köln (DE); Werner, Sabine, 40227 Düsseldorf (DE)

(57) **Abstract**

The invention relates to a rapid method for isolating nucleic acid from a nucleic acid source, wherein the nucleic acid source is lysed in the absence of a chaotropic salt and in the absence of an alcohol. The lysate is subsequently filtered through a porous matrix consisting of a material based on silica or of a silica coated material, which binds the nucleic acid in the absence of a chaotropic salt and in the absence of an alcohol. Finally, the nucleic acid is eluted from the porous matrix by an aqueous buffer solution. Furthermore, this invention relates to a test kit in order to isolate the nucleic acid.

## Description

The invention relates to a rapid method for isolating nucleic acid from a nucleic acid source, wherein the nucleic acid source is lysed in the absence of a chaotropic salt and in the absence of an alcohol.

From the state of the art methods for isolating nucleic acid from complex starting materials, e.g. whole blood, biopsies, plant tissue, etc., are known. These methods usually comprise lysis of biological material by a detergent in the presence of protein degrading enzymes, followed by several extractions with organic solvents, e.g., phenol and/or chloroform, ethanol precipitation and dialysis of the nucleic acids. This kind of methods for isolating DNA are very laborious and time-consuming. The relatively large number of steps required to purify nucleic acid from such starting materials increase the risk of transmission of nucleic acid from sample to sample in the simultaneous processing of several different samples. When the nucleic acid is isolated for the subsequent detection of the presence of specific nucleic acid sequences, e.g. genotyping or the like, by means of a nucleic acid amplification method, for example the utmost sensitive polymerase-chain-reaction (PCR), the increased risk of such a transmission of nucleic acid between different samples which causes false positive results is a serious drawback.

Methods for isolating nucleic acid from a complex starting material were simplified in time. These methods often included the use of chaotropic salts, e.g. guanidinium chloride, and/or the use of alcohols, e.g. ethanol or isopropanol. Boom et al. (US 5,234,809) described a method, wherein the nucleic acid originating from a nucleic acid containing starting material is bound to a nucleic acid binding material in the presence of a chaotropic salt.

The problem underlying the present invention is to provide a simple method for isolating nucleic acid, which is both rapid and not depending on hazardous and expensive compounds, i.e. reagents like chaotropic salts and/or alcohols.

The present invention solves this problem by providing a rapid as well as a low cost method for isolating nucleic acid. The method according to the invention is advantageously designed to work without an alcohol and without a chaotropic salt, which both are high cost factors. Furthermore, the method according to the invention is a very rapid method for isolating nucleic acid. Therefore, the method according to the invention is exceptionally useful, e.g., in normally time- and cost-intensive high throughput screening series.

In particular, the invention relates to a simple nucleic acid isolation procedure from a nucleic acid source by lysing the nucleic acid source in the absence of an alcohol and the absence of a chaotropic salt. The resulting lysate is optionally centrifuged at appropriate G force to eliminate cell debris from the lysate. Subsequently, the lysate - or in case of a centrifugation step the supernatant of the centrifuged lysate - is filtered by moving the lysate through a porous matrix consisting of a material based on silica or of a silica coated material, whereby the nucleic acid binds to the porous matrix in the absence of a chaotropic salt and in the absence of an alcohol. One or more optional washing steps may follow the filtration step. Finally, the nucleic acid is eluted from the porous matrix by using a suitable elution buffer.

In a preferred embodiment the nucleic acid isolated by the method according to the invention serves as a template in a subsequent application like AFLP, RFLP, microsatellite analysis, southern blot, PCR, quantitative real-time PCR or the like, even more preferably in a PCR or quantitative real-time PCR application. This makes the method according to the invention a useful tool, e.g., in rapid and economic genotyping or in otherwise cost-intensive high throughput genetic screenings.

The term 'nucleic acid' as used herein stands for DNA and RNA as well as hybrids thereof. In a preferred embodiment the nucleic acid is DNA. In an even more preferred embodiment the nucleic acid is genomic DNA. Usually, the nucleic acid isolated by the method according to this invention has a size ranging from about 10 kbp to about 50 kbp.

The term 'nucleic acid source' as used herein stands for any kind of biological tissue or cell material, e.g. mammalian cells, organs, biopsies, blood, serum, muscle, bone marrow, bacteria, yeast, any sort of plant tissue or cells, like seeds or leaves, etc. The following non-limiting embodiments shall demonstrate the range of application of the method according to the invention. A typical example is the screening of a series of individuals for a transgene, e.g. transgenic animals like mice or the like, comprising isolating the genomic DNA from a tissue sample and a subsequent PCR with transgene specific primers or a subsequent southern blot analysis or a subsequent RFLP analysis. Another typical example is a genetical screening of seeds along the lines of the above mentioned example. Yet another example is the isolation of DNA from a whole blood sample. Combined with a subsequent, e.g., PCR it is a low cost and powerful tool to rapidly detect, e.g., genetical disorders.

The lysing of the nucleic acid source can be performed - depending on the kind of nucleic acid source - by any suitable method as known from the art. Therefore, e.g. homogenization of the nucleic acid source frozen in liquid nitrogen, e.g. in a MixerMill (Retsch, Haan, Germany), as well as gentle lysis, e.g. by using a lysis buffer comprising a detergent, e.g. SDS, is suitable. According to the invention, the lysing buffer does not contain an alcohol and does not contain a chaotropic salt. Numerous suitable lysing buffers are known in the art. Typically - but not limited to - a lysing buffer comprises, e.g., a detergent, e.g. SDS, preferably in a concentration of from 0,1 % (w/v) to 5 % (w/v), and/or Triton X-100, preferably in a concentration of from 1 % (v/v) to 20 % (v/v), and/or Tween 20, preferably in a concentration of from 1 % (v/v) to 5 % (v/v), and/or NP40 (Nonidet® P40), preferably in a concentration of from 1 % (v/v) to 5 % (v/v), and/or sarcosyl, preferably in a concentration of from 0,1 % (v/v) to 4 % (v/v); and/or a chelator, e.g. EDTA, preferably in a concentration of from 5 mM to 200 mM; and/or other suitable reagents, e.g. Tris, preferably in a concentration of from 10 mM to 30 mM and at a pH of from 7,0 to 9,0, and/or urea, preferably in a concentration of from 0,1 M to 7 M, and/or CTAB (Cetyltrimethylammonium bromide), preferably in a concentration of from 1 % (w/v) to 2 % (w/v), and/or PVP (polyvinylpyrrolidone), preferably in a concentration of from 0,1 % (w/v) to 2 % (w/v), and/or β-mercaptoethanol, preferably in a concentration of from 50 mM to 150 mM, and/or lithium chloride, preferably in a concentration of from 0,1 M to 3 M, and/or sodium acetate, preferably in a concentration of from 10 mM to 150 mM, and/or sodium chloride, preferably in a concentration of from 0,1 M to 5 M and/ or other salts, e.g., potassium chloride and/or ammonium chloride in molar ranges, and/or enzymes, e.g., proteolytic enzymes and/or lysozyme, or the like. Some non-limiting examples of lysing buffers are given in table 1.

**Table 1:**

| Non-limiting examples for suitable lysing buffers according to the invention | | |
|---|---|---|
| 10 mM Tris/HCl pH 8.0 | 200 mM Tris/HCl pH 7.5 | 10 mM Tris/HCl pH 8.0 |
| 10 mM EDTA | 250 mM NaCl | 50 mM EDTA |
| 2% (w/v) SDS | 25 mM EDTA | 0,5 M NaCl |
| | 0,5 % (w/v) SDS | 2% (w/v) PVP |
| 50 mM Tris/HCl pH 8.0 | 50 mM Tris/HCl pH 8.0 | 100 mM sodium acetate pH 4.8 |
| 100 mM EDTA | 20 mM EDTA | 50 mM EDTA |
| 0,5% (w/v) sarcosyl | 0,1 M NaCl | 500 mM NaCl |
| 100 µg/ml proteinase K | 1% (w/v) PVP | 1.5% (w/v) SDS |
| 50 mM KCI | 2 % (w/v) CTAB | 2 % (w/v) CTAB |
| 1,5 mM MgCl2 | 50 mM Tris/HCl pH 8.0 | 100 mM Tris/HCl pH 8.0 |
| 10 mM Tris pH 8.5 | 0,7 M NaCl | 1,4 M NaCl |
| 0,01% (w/v) gelatine | 10 mM EDTA | 50 mM EDTA |
| 0,45 % (v/v) NP-40 | 100 mM ß-mercaptoethanol | 50 mM ß-mercaptoethanol |
| 0,45 % (v/v) Tween 20 | | |
| 100 µg/ml proteinase K | | |
| 200 mM Tris pH 7.5 | 50 mM Tris/HCl pH 9.0 | 100 mM Tris/HCl pH 8.0 |
| 250 mM NaCl | 5 mM EDTA | 50 mM EDTA |
| 25 mM EDTA | 150 mM LiCl | 0,5 M.NaCl |
| 0,5 % (w/v) SDS | 5 % SDS | 10 mM ß-mercaptoethanol |
| 200 mM Tris/HCl pH 7.5 | 100 mM NaCl | 4 M urea |
| 25 mM EDTA | 50 mM Tris/HCl pH 8,2 | 200 mM Tris/HCl pH 7.4 |
| 250 mM NaCl | 100 mM EDTA | 20 mM NaCl |
| 0,5% (w/v) SDS | 3 % (w/v) SDS | 200 mM EDTA |
| 7 M urea | 100 mM NaCl | 100 mM Tris/HCl pH 9.5 |
| 5 mM EDTA pH 8.0 | 0.5% (v/v) sarcosyl | 10 mM EDTA |
| 2% (w/v) SDS | | 1 M KCI |
| 2,5 M LiCl | 100 mM sodium acetate pH 5.4 | 50 mM EDTA |
| 50 mM Tris/HCl pH 8.0 | 50 mM EDTA | 50 mM Tris/HCl pH 8.0 |
| 62,5 mM EDTA | 0,5 M NaCl | |
| 4 % (v/v)Triton X-100 | 2 % (w/v) PVP | |
| 1 % (w/v) CTAB | 2 % (w/v) CTAB | 320 mM sucrose |
| 50 mM Tris/HCl pH 8.0 | 100 mM Tris/HCl pH 8.6 | 50 mM Tris/HCl pH 7.5 |
| 0,7 M NaCl | 1,4 M NaCl | 5 mM MgCl₂ |
| 10 mM EDTA | 20 mM EDTA | 1 % (v/v) Triton X-100 |
| 150 mM ß-mercaptoethanol | 1 % (w/v) PVP | |
| 100 mM Tris/HCl pH 8.0 | 29 mM Tris/HCl pH 8.0 | 100 mM Tris/HCl pH 7.5 |
| 100 mM EDTA | 2 mM EDTA | 50 mM EDTA |
| 10 mM NaCl | 1,2 % (v/v) Triton X-100 | 0,5 M NaCl |
| 1% (w/v) SDS | optionally: | 10 mM ß-mercaptoethanol |
| optionally: | lysozyme/lysostaphin/proteinase K | 1 % (w/v) SDS |
| 100 µg/ml proteinase K | | |

Depending on the kind of nucleic acid source, the nucleic acid source/lysis buffer-mixture shall be allowed to incubate for an appropriate time at an appropriate temperature. Suitable lysis protocols are well known to those skilled in the art and depend on the kind of nucleic acid source used.

The centrifugation step subsequent to the lysing procedure and prior to the filtration step is an optional step to avoid clogging of the porous matrix material by cell debris. The centrifugation step is performed at appropriate conditions, i.e. appropriate temperature and appropriate G force, which are well known to those skilled in the art. Typically, but not limited to, the centrifugation is performed at a temperature in a range from about 4°C to about room temperature and at a G force ranging from about 1000 x g to about 6000 x g.

To enhance the performance of the method according to the invention one or more enzymes may optionally be added to the lysing step, the filtration step, the elution step or to the isolated nucleic acid resulting from the elution step, for example, but not limited to enzymes with a RNase activity and/or a protease activity. The protease will, e.g., enhance the lysis performance and/or will eliminate DNase activity depending on the kind of nucleic acid source and the chosen lysis method. The RNase will, e.g., reduce RNA contamination of the isolated DNA and, hence, enhance the performance of a subsequent PCR. Typically, a protease or a RNase is added during the lysis step. Furthermore, lysozyme, which helps to lyse bacterial cell walls, may be a useful addition to one or more of the steps. The handling of such enzymes is well known to those skilled in the art.

The filter material used in the method according to the invention is a porous matrix consisting of a material based on silica or of a silica coated material. The matrix may be composed of particles of any size as well as of a one-piece membrane. In a preferred embodiment the porous matrix material is a porous silica membrane. Preferably, the porous matrix material possesses a siliceous oxide coated surface. The term 'porous' as used herein means that the matrix material comprises pores having the size of ranging from 0,2 µm to 3,2 µm, preferably from 0,3 µm to 2,8 µm and even more preferably from 0,5 µm to 2,0 µm. In a preferred embodiment, the porous matrix material is a membrane embedded in a single column filter tube or - in another preferred embodiment - is integrated in a multi-well filter plate, preferably a 96-well filter plate or a 384-well filter plate. Within these supporting materials, the membrane can be assembled in one or more layers, wherein the pore size of one layer may differ from the pore size of the other layer(s). The lysate - or in case of a centrifugation step the supernatant of the centrifuged lysate - is filtered by moving the lysate through the porous matrix material, e.g. by centrifugation or vacuum or the like.

The one or more optional washing steps subsequent to the filtration step and prior to the elution step may be performed by using any suitable washing buffer. Numerous suitable washing buffers are well known to those skilled in the art. As well known from the state of the art, appropriate buffers comprising an alcohol, e.g. ethanol and/or isopropanol, may be suitable for the washing steps. Suitable washing buffers may comprise - but not limited to -, e.g., sodium chloride, preferably in a concentration of from 10 mM to 150 mM, and/or Tris, preferably in a concentration of from 10 mM to 30 mM and a pH of from 7,0 to 9,0, and/or ethanol, preferably in a concentration of from 10 % (v/v) to 100 % (v/v), and/or isopropanol, preferably in a concentration of from 25 % (v/v) to 100 % (v/v), and/or Tween 20, preferably in a concentration of from 0,1 % (v/v) to 3 % (v/v), and/or lithium chloride, preferably in a concentration of from 100 mM to 500 mM, or the like. Some non-limiting examples of lysing buffers are given in table 2.

**Table 2:**

| Non-limiting examples for suitable washing buffers according to the invention | |
|---|---|
| 100 mM NaCl | 25 mM Tris |
| 10 mM Tris/HCl pH 7,5 | 100 mM potassium acetate |
| 70 % (v/v) ethanol | 10 mM magnesium acetate |
| | pH 7,8 |
| 0,5 M NaCl | 0,4 M LiCl |
| 5 %(w/v) PEG 8000 | 77,7 % (v/v) ethanol |
| 5 M NaCl | 10 mM Tris/HCl pH 7,5 |
| | 80 % (v/v) ethanol |
| 10 mM Tris/HCl pH 7,5 | 0,9 M potassium acetate |
| 25 mM NaCl | 70 % (v/v) ethanol |
| 80 % (v/v) ethanol | |
| 96 % (v/v) ethanol | 100 % (v/v) isopropanol |

The elution of the nucleic acid from the porous silica material can be performed by using any suitable buffer. Numerous elution buffers are known from the art and are obvious to those skilled in the art. The elution buffer is typically, but not limited to, a low salt buffer and/or low molar Tris buffer. Suitable elution buffers may comprise - but not limited to -, e.g., Tris, preferably in a concentration of from 1 mM to 15 mM and a pH of from 7,5 to 9,0, and/or EDTA, preferably in a concentration of from 0,1 mM to 2 mM. Elution may also be performed with distilled water. Non-limiting examples are, e.g., 10 mM Tris/HCl with a pH ranging from 8,0 to 9,0, optionally combined with 0,5 mM to 1 mM EDTA.

Furthermore, the invention relates to a test kit to perform an isolation of nucleic acid from a nucleic acid source by the method according to the invention. This test kit shall contain at least a) porous matrix consisting of a material based on silica or of a silica coated material, b) a lysis buffer not containing a chaotropic salt and not containing an alcohol and c) an elution buffer.

### Examples

### Example 1: Isolation of genomic DNA from mouse heart

Mouse heart tissue (10 mg per preparation) was mixed with 180 µl of lysis buffer (3% (w/v) SDS; 100 mM NaCl; 100 mM EDTA; 50 mM Tris/HCl; pH 8,3) and 20 µl proteinase K (600 AU/ml) (QIAGEN, Hilden, Germany). The mixture was incubated for 24 hours at 55°C. Subsequently, 200 µl of the lysate were vortexed followed by a centrifugation step (5000 x g, 2 min). The supernatant was loaded onto a spin column comprising a micro filter column molding with a standard polypropylene frit, which was assembled by inserting two 7,5 mm diameter round punches of a Whatman GF/B Glass Microfiber membrane, and, subsequently, filtered by centrifugation. The filtration step was followed by two washing steps each with 100 µl washing buffer (100 mM NaCl; 10 mM Tris HCl; 70% (v/v) ethanol; pH 5). The elution step was performed with 100 µl buffer (0,5 mM EDTA; 10 mM Tris/HCl; pH 9). 15 µl of the eluate were loaded onto an agarose gel. The gel clearly showed distinct bands of genomic DNA in a size ranging from about 10 kbp to about 50 kbp.

### Example 2: Isolation of genomic DNA from wheat

Wheat (50 mg per preparation) was grinded to a fine powder using a MixerMill MM 300 (Retsch, Haan, Germany) and subsequently resuspended in 200 µl of lysis buffer (1,4% (w/v) SDS; 2% (w/v) polyvinylpyrrolidone; 500 mM NaCl; 100 mM sodiumacetate; 50 mM EDTA; pH 5,5) and 1 µl RNase A (7000 U/ml) (QIAGEN, Hilden, Germany). The mixture was subsequently vortexed and centrifuged for 2 min at 5000 x g. The supernatant was loaded onto a spin column comprising a micro filter column molding with a standard polypropylene frit, which was assembled by inserting two 7,5 mm diameter round punches of a Whatman GF/B Glass Microfiber membrane, and, subsequently, filtered by centrifugation. The filtration step was followed by two washing steps each with 100 µl washing buffer (100 mM NaCl; 10 mM Tris HCl; 70% (v/v) ethanol; pH 5). The elution step was performed with 100 µl buffer (0,5 mM EDTA; 10 mM Tris/HCl; pH 9). 15 µl of the eluate were loaded onto an agarose gel. The gel clearly showed distinct bands of genomic DNA in a size ranging from about 10 kbp to about 50 kbp.

## Claims

1. A method for rapidly isolating nucleic acid from a nucleic acid source comprising the steps of:
a) lysing the nucleic acid source,
b) filtering the lysate through a porous matrix consisting of a material based on silica or of a silica coated material to bind the nucleic acid to the porous matrix in the absence of an alcohol and in the absence of a chaotropic salt,
c) eluting the nucleic acid from the porous matrix of step b) by using an aqueous buffer solution.

2. A method according to claim 1, wherein the nucleic acid is DNA.

3. A method according to claim 2, wherein the DNA is genomic DNA.

4. A method according to claim 1 to 3, wherein the nucleic acid is of a size ranging from about 10 kbp to about 50 kbp.

5. A method according to claim 1, wherein the nucleic acid source is any sort of biological tissue or cell material.

6. A method according to claim 5, wherein the nucleic acid source is mammalian cells, organs, biopsies, blood, serum, muscle, bone marrow, bacteria, yeast, and/or any sort of plant tissue or cells, like seeds or leaves.

7. A method according to claim 1, wherein the nucleic acid source is lysed using a buffer not containing a chaotropic salt and not containing an alcohol.

8. A method according to claim 1, wherein a RNase and/or a protease and/or lysozyme is added to one or more of the steps of claim 1.

9. A method according to claim 1, wherein the porous matrix comprises a siliceous oxide coated surface.

10. A method according to claim 1 or 9, wherein the porous matrix is a porous silica membrane.

11. A method according to claim 1, 9 or 10, wherein the porous matrix comprises pores having the size ranging from 0,2 µm to 3,2 µm.

12. A method according to claim 11, wherein the porous matrix comprises pores having the size ranging from 0,3 µm to 2,8 µm.

13. A method according to claim 12, wherein the porous matrix comprises pores having the size ranging from 0,5 µm to 2,0 µm.

14. A method according to claim 1, wherein the isolated nucleic acid serves as a template in a subsequent application like AFLP, RFLP, microsatellite analysis, southern blot, PCR or quantitative real-time PCR.

15. A method according to claim 14, wherein the isolated nucleic acid serves as a template in a subsequent PCR or subsequent quantitative real-time PCR application.

16. A method according to claim 1, wherein the lysate of step a) of claim 1 is centrifuged to eliminate cell debris from the lysate prior to step b) of claim 1.

17. A method according to claim 1, wherein one or more washing steps are performed subsequent to step b) of claim 1 and prior to step c) of claim 1.

18. A method according to claim 17, wherein the washing step is performed using a washing buffer.

19. A method according to claim 1, wherein the porous matrix of step b) of claim 1 is a membrane embedded in a single column filter tube.

20. A method according to claim 1, wherein the porous matrix of step b) of claim 1 is a membrane integrated in a multi-well filter plate.

21. A method according to claims 19 and 20, wherein the membrane is assembled in one or more layers.

22. A method according to claim 21, wherein the pore size of one layer differs from the pore size of the other layer(s).

23. A kit for performing the method according to claims 1 to 22 comprising at least:
a) a porous matrix consisting of a material based on silica or of a silica coated material
b) a lysing buffer containing no alcohol and containing no chaotropic salt
c) an elution buffer.
